# EUROPÄISCHE PATENTANMELDUNG

(11) **EP 1 084 685 A1**
(43) Veröffentlichungstag der Anmeldung: **21.03.2001**
(21) Anmeldenummer: 00119572.6
(22) Anmeldetag: 07.09.2000
(51) Int. Cl.: A61F 2/01

(54) **Gefässfiltersystem**

(30) Priorität: 14.09.1999 DE 29916162 U
(71) Anmelder: Cormedics GmbH, 82041 Deisenhofen (DE)
(72) Erfinder: Braun, Michael, 71522 Backnang (DE); Geis, John S., 82031 Grünwald (DE)
(74) Vertreter: Kador & Partner

(57) **Zusammenfassung**

Die vorliegende Erfindung umfaßt ein Gefäßfiltersystem für die nichtpermanente Anwendung, das mit einen Führungsdraht versehen ist, einer Filtermembran, die einen im Bereich des proximalen Endes des Führungsdrahtes angebrachten inneren Bereich und einen freien äußeren Bereich aufweist, und eine Schirmstruktur, die zumindest am äußeren Bereich der Filtermembran vorgesehen ist, aus einem flexiblen Material besteht und das Öffnen und Schließen des durch die Schirmstruktur und die Filtermembran gebildeten Schirmes zuläßt, dadurch gekennzeichnet, daß das System berührungslos wirkende Mittel zum Schließen des Schirmes aufweist. Diese Mittel können magnetischer Natur sein oder auf Mechanismen, die auf Temperaturänderungen basieren, beruhen.

## Beschreibung

Die Erfindung betrifft ein Gefäßfiltersystem, insbesondere ein Gefäßfiltersystem, das einen Führungsdraht, eine Filtermembran und eine Schirmstruktur aufweist, wobei die Filtermembran einen inneren Bereich, der im Bereich des proximalen Endes des Führungsdrahtes angebracht ist, sowie einen freien äußeren Bereich aufweist und die Schirmstruktur aus einem flexiblen Material besteht und das Öffnen und Schließen des durch die Schirmstruktur und die Filtermembran gebildeten Schirmes zuläßt.

Es wurden eine Vielzahl, beispielsweise angioplastischer, operative und nicht-operative Verfahren entwickelt, in denen Ablagerungen in Blutgefäßen entfernt werden. Bei diesen Verfahren, sowie auch bei der Implantation von By-passes an den mit Stenosen betroffenen Stellen, besteht die Gefahr, daß sich Teile des Belages oder Thromben lösen und in einer Embolie enden können. Um solche Gefahren verhindern zu können wurden Gefäßfilter entwickelt, die Mikro- und Makro-Partikel blockieren sollen.

Ein solcher Gefäßfilter ist beispielsweise in WO 98/33443 beschrieben. Dieser herkömmliche Gefäßfilter wird durch einen Aufspann-Mechanismus gebildet. Der Filter ist hierbei in seiner aufgespannten Position im wesentlichen senkrecht zu der Achse des Führungsdrahtes, über den er in das Blutgefäß eingeführt werden kann. Zum Entfernen aus dem Blutgefäß sehen die herkömmlichen Gefäßfilter Rückholfäden vor. Diese können an dem äußeren Umfang des Filters befestigt sein und werden über einen Draht, der beispielsweise im Führungsdraht geführt werden kann, betätigt. Diese Einholen des Filters, z.B. nach Beendigung des Eingriffes, erfordert das Aufbringen einer ausreichenden Kraft über den Draht, der mit den Rückholfäden verbunden ist. Da in den meisten Verfahren die Stelle über die der Filter in das Gefäß eingeführt wird weit von dem Einsatzort des Filters liegt, ist die Länge des Führungsdrahtes und des Rückholdrahtes erheblich. Bei Eingriffen an der Halsschlagader wird der Filter beispielsweise meist über die Arterie in der Leiste eingeführt. Hierbei kann der Führungsdraht und damit auch der Rückholdraht Längen von mehr als einem Meter aufweisen. Um die zum Einholen des Filters notwendigen Kräfte in einer Entfernung dieser Größe aufbringen zu können müssen die Kräfte die auf den Rückholdraht ausgeübt werden groß sein.

Aufgrund dieser großen Kräfte und der erheblichen Länge, sowie der Tatsache, daß der Führungsdraht dem Verlauf des Blutgefäßes folgen muß und daher nicht gerade durch den Körper geführt werden kann, kann es bei herkömmlichen Gefäßfiltersystemen zum Verhaken des Führungsdrahtes sowie des Rückholdrahtes und der Rückholfäden kommen. Durch dieses Verhaken kann der Vorgang des Rückholens bzw. des Zusammenklappen und Entfernen des Filters erheblich behindert und verlängert werden. Dadurch verlängert sich die Zeit des gesamten Eingriffes, was für den Patienten, insbesondere aufgrund benötigter Narkotika bedenklich sein kann. Zudem können durch verkantete Drähte Verletzungen des Blutgefäßes entstehen.

Der Erfindung liegt daher die Aufgabe zugrunde ein Gefäßfiltersystem zu schaffen, daß die Nachteile des Standes der Technik nicht aufweist insbesondere leicht und zuverlässig zu handhaben ist und die Gefahr der Verletzung des Patienten und die Belastung durch längere Eingriffsdauern herabsetzt.

Der Erfindung liegt die Erkenntnis zugrunde, daß diese Aufgabe durch ein Gefäßfiltersystem gelöst werden kann, bei dem entweder kein oder nur ein sehr geringer Kraftaufwand über die Länge zwischen Ort des Eintrittes in den Körper und dem Ort des Filters notwendig ist, um den Filter zu schließen.

Die Aufgabe wird erfindungsgemäß durch ein Gefäßfiltersystem gemäß dem Oberbegriff des Anspruchs 1 gelöst, wobei dieses System berührungslos wirkende Mittel zum Schließen des Schirmes aufweist.

Das erfindungsgemäße Gefäßfiltersystem wird vorzugsweise für die nichtpermanente Anwendung eingesetzt.

Das erfindungsgemäße System weist den Vorteil auf, daß der Aufbau des Schirmes einfach sein kann. Es sind keine mechanischen Verbindungen zwischen der Filtermembran bzw. der Schirmstruktur zum einen und einem Element, das von der Position des Filters zu der Eintrittsstelle des Filtersystems in den Körper reicht, zum anderen notwendig. Weiterhin kann es aufgrund dieses einfachen Aufbaus nicht zum Verhaken, Verkanten oder zum Abreißen der mechanischen Verbindung kommen. Hierdurch wird das Risiko für den Patienten weiter herabgesetzt. Schließlich können berührungslos wirkende Mittel von außen aktiviert werden ohne, daß ein großer Kraftaufwand über die Länge zwischen der Position des Filters und der Eintrittsstelle des Filtersystems in den Körper erforderlich ist. Dadurch können auch Verletzungen oder das Verhaken von Führungsdraht und Rückholelement vermieden werden.

Die Schirmstruktur besteht bei dem erfindungsgemäßen Gefäßfiltersystem aus einem flexiblen Material, das das Öffnen und Schließen der Schirmstruktur erlaubt. Bevorzugt stellt die Schirmstruktur daher eine Feder auf, die im entspannten Zustand den aufgespannten Schirm stützt. Die Feder kann durch ein beispielsweise zick-zack-förmig gebogenes, flexibles, drahtförmiges Material, durch eine Spiralfeder oder auch durch ein mit einer Spiralfeder überzogenes flexibles drahtförmiges Material dargestellt werden. Durch Aktivierung des Materials der Schirmstruktur wird diese zusammen gezogen und die Feder damit gestaucht. Die Federkraft der Schirmstruktur wird vorzugsweise so gewählt, daß zum einen ein ausreichender Druck gegen die Wand des Blutgefäßes im aufgespannten Zustand erzeugt wird und zum anderen die Kraft zum Zusammenklappen bzw. Schließen des Schirms so gering ist, daß diese durch berührungslos wirkende Mittel erbracht werden kann.

Gemäß einer Ausführungsform der Erfindung ist das Mittel zum Schließen durch das Material der Schirmstruktur gegeben. Die Schirmstruktur ist zumindest am äußeren Bereich der Filtermembran vorgesehen. Wird das Material dieser Struktur so gewählt, daß es sich unter gewissen Umständen zusammenzieht, so wird dadurch das Schließen des Schirmes bewirkt. Bei dieser Ausführungsform können beispielsweise Materialien eingesetzt werden, die sich durch chemische oder physikalische Aktivierung zusammenziehen und so das Schließen des Schirmes bewirken. Da die Aktivierung im Blutgefäß, d.h. im Körper stattfindet müssen Materialien gewählt werden deren Aktivierung unbedenklich ist. Es können Aktivierungen durch Einführen von Medien in das Blut im Bereich des Filters eingesetzt werden. Weiterhin können solche Materialien verwendet werden, die sich durch die Einwirkung von Strahlungen zusammen ziehen.

Gemäß einer weiteren Ausführungsform ist zusätzlich ein Mittel zum Schließen auf dem Führungsdraht vorgesehen. Dieses Mittel kann mit dem durch das Material der Schirmstruktur gegebenen Mittel zusammenwirken. Hierbei kann die zum Zusammenziehen der Schirmstruktur notwendige Aktivierung des Materials der Schirmstruktur durch das auf dem Führungsdraht vorgesehene Mittel erzeugt werden. Das Mittel kann entweder unmittelbar auf dem Führungsdraht befestigt sein, oder verschiebbar darauf angeordnet sein.

Im ersten Fall ist das Mittel vorzugsweise in dem Bereich des Führungsdrahtes angeordnet, an dem die Schirmstruktur nach dem Zusammenklappen anliegt. Im zweiten Fall kann das Mittel beispielsweise auf einem über den Führungsdraht geschobenen, d.h. von diesem getragenen, Katheter befestigt sein. Es ist bei dieser Ausführungsform nur notwendig das Mittel von einer Position in einer gewissen Entfernung von dem Schirm in die Position zu bringen, in der es mit der Schirmstruktur zusammenwirken kann und das Schließen des Schirmes bewirkt wird. Die Strecke über die der Katheter verschoben werden muß ist daher sehr klein und insbesondere geringer als die Strecke um die ein Rückholdraht nach den Ausführungsformen des Standes der Technik verschoben werden muß. Zudem muß bei dem Verschieben des erfindungsgemäß verwendeten Katheters lediglich die Kraft zum Bewegen des Katheters als solchen aufgebracht werden. Diese Kraft ist deutlich geringer als jene die zum mechanischen Zusammenklappen des Schirmes über Rückboldraht und Rückholfäden entgegen dem Blutstrom notwendig ist.

Gemäß einer bevorzugten Ausführungsform ist das auf dem Führungsdraht vorgesehene Mittel ein Magnetkatheter. Bei dieser Ausführungsform kann der Magnetkatheter auf den Führungsdraht aufgefädelt sein. Der Magnet ist hierbei an dem, dem proximalen Ende des Führungsdrahtes zugewandten, Ende des Magnetkatheters angebracht. Es kann sich um einen Permanent-Magneten oder aber auch um einen zeitweise aktivierbaren, z.B. einen Elektro-Magneten handeln. Bei dieser Ausführungsform dient der Magnetkathether als Verschiebungselement für den Magneten als solchen und erlaubt somit die exakte Positionierung des Magneten bezüglich der Position des Filters.

Gemäß einer weiteren bevorzugten Ausführungsform ist das Material der Schirmstruktur durch Temperaturänderungen aktivierbar, d.h. zieht sich bei Temperaturänderung zusammen und bewirkt so das Schließen des Schirmes. Bevorzugt wird bei dieser Ausführungsform die Schirmstruktur aus einer Nitinol-Feder gebildet. Dieses Material das eine Ti-Ni-Legierung darstellt besitzt den Vorteil, daß es sich zum einen bei Kälte zusammenzieht und zum anderen ein Formgedächnis aufweist, d.h. einen sogenannten Memory-Effekt zeigt. Der Schirmstruktur kann daher die Form, in der der Schirm das zu behandelnde Blutgefäß in seinem Querschnitt optimal bedeckt, vorgegeben werden und ein Zusammenziehen der Schirmstruktur entgegen diesem Formgedächnis durch kurzfristiges einbringen eines kalten Mediums, beispielsweise Eiswasser erzielt werden.

Gemäß einer weiteren Ausführungsform ist das Material der Schirmstruktur magnetisierbar. Durch diese Beschaffenheit des Materials der Schirmstruktur ist die Aktivierung des Materials durch ein auf dem Führungsdraht vorgesehenes Mittel durch Magnetismus möglich. Weiterhin ist es möglich die Schirmstruktur so auszubilden, daß sich vom inneren, am Führungsdraht befestigten, Bereich der Filtermembran zum äußeren Bereich der Filtermembran Stege aus magnetisierbarem Material radial erstrecken. Die Stege können zum Öffnen des Schirmes gleich gepolt sein, wodurch sie sich gegeneinander abstoßen und zum Schließen alternierend gepolt werden, wodurch sie sich gegenseitig anziehen.

In einer bevorzugten Ausführungsform weist das Filtersystem eine Führungshülse zur Aufnahme des Führungsdrahtes und des Schirmes auf. Diese Führungshülse kann zum einen dazu dienen daß der Schirm in das Blutgefäß eingeführt werden kann ohne eine Verletzung des Blutgefäßes zu verursachen, zum anderen dient die Führungshülse beim Entfernen des Filters als Halterung für den geschlossenen Schirm. Dieser wird nach dem Vorgang des Schließens vorzugsweise über den Führungsdrabt in die Hülse zurückgezogen und kann sich bei geeigneter Wahl des Durchmessers der Führungshülse nicht oder nur geringfügig wieder ausdehnen. Hierdurch kann auch nach dem das Mittel zum Schließen deaktiviert ist, z.B. der Magnet abgeschaltet, oder die Zugabe von Eiswasser gestoppt ist, der Schirm in der zusammengeklappten Position gehalten werden, ohne daß es weiterer Vorrichtungen bedarf.

Die Führungshülse kann zudem als Leitung für Medien dienen, die in den Bereich des Schirmes gebracht werden sollen, um die Aktivierung des Materials der Schirmstruktur, d.h. deren Zusammenziehen zu bewirken, z.B. als Leitung für Eiswasser.

Die Erfindung wird im Folgenden anhand der beiliegenden Zeichnung, die mögliche Ausführungsformen der Erfindung darstellen, den Schutzbereich aber nicht beschränken, weiter beschrieben.

Es zeigen:
Figur 1: eine Ausführungsform des erfindungsgemäßen Gefäßfiltersystems in seiner Anwendung;
Figur 2: schematische Darstellung einer Ausführungsform;
Figur 3a: schematische Darstellung einer Ausführungsform der Erfindung mit Magnetkatheter mit aufgespanntem Schirm;
Figur 3b: schematische Darstellung einer Ausführungsform der Erfindung mit Magnetkatheter mit geschlossenem Schirm;
Figur 4a: schematische Darstellung einer weiteren Ausführungsform der Erfindung mit aufgespanntem Schirm;
Figur 4b: schematische Darstellung einer weiteren Ausführungsform der Erfindung mit geschlossenem Schirm;
Figur 5a: schematische Darstellung einer weiteren Ausführungsform der Erfindung mit Magnetkatheter mit aufgespanntem Schirm;
Figur 5b: schematische Darstellung einer weiteren Ausführungsform der Erfindung mit Magnetkatheter mit geschlossenem Schirm;
Figur 6a: schematische Darstellung einer weiteren Ausführungsform der Erfindung mit Führungshülse im geschlossenem Zustand;
Figur 6b: schematische Darstellung einer weiteren Ausführungsform der Erfindung mit Führungshülse im aufgespannten Zustand.

In den einzelnen Figuren sind entsprechende Bestandteile mit den gleichen Referenznummern bezeichnet.

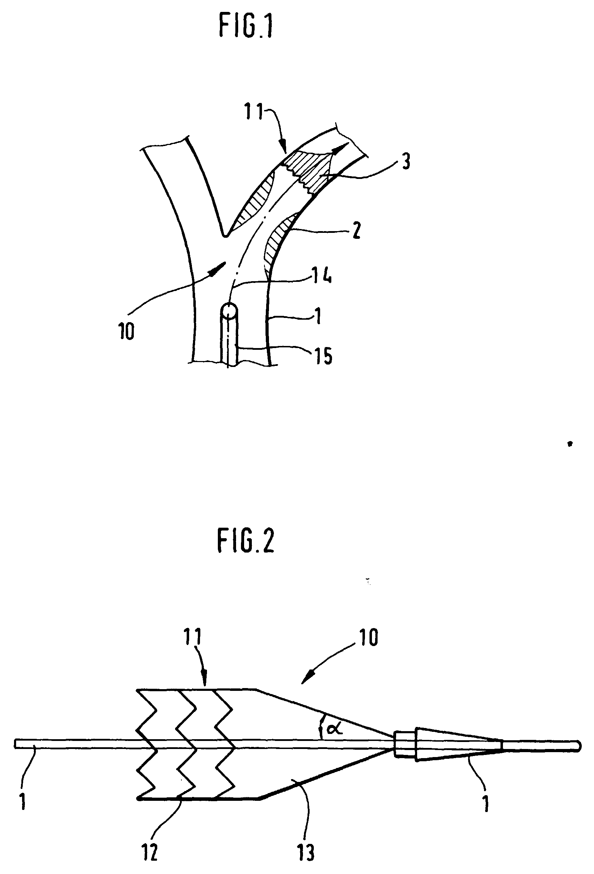

In Figur 1 ist ein Gefäßfiltersystem 10 dargestellt, das in ein Blutgefäß 1 eingebracht ist. Der Schirm 11 ist in dem Blutgefäß 1 so aufgespannt, daß er diese über seinen Querschnitt abdeckt und an der Wand des Blutgefäß 1 über einen Bereich anliegt. Unterhalb des Schirmes 11 sind Stenosen 2 angedeutet. Diese Stenosen, die zu einer Verengung des Blutgefäßes führen, sollen durch die Verfahren, in denen das erfindungsgemäße Gefäßfiltersystem 10 eingesetzt wird, entfernt werden. Beim Entfernen dieser Stenosen lösen sich Partikel (Thromben) 3, die vom Blutstrom erfaßt und mitgerissen werden. In Figur 1 ist dargestellt, daß diese Thromben in dem Schirm gefangen werden. Der Schirm ist an einem Führungsdraht 14 befestigt. Dieser Führungsdraht erstreckt sich in der in Figur 1 dargestellten Position über das Ende einer Führungshülse 15 hinaus und ist in dieser geführt.

In Figur 2 ist der Aufbau eines erfindungsgemäßen Gefäßfiltersystems 10 schematisch dargestellt. Der Führungsdraht 14 besitzt ein proximales Ende, das mit einem sogenannten 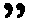 Tip" 141 versehen ist. Dieser Aufsatz 152 dient dem leichteren Einführen des Gefäßfiltersystems 10 und verhindert Verletzungen des Blutgefäßes beim Einführen. Anschließend an den Aufsatz 141 ist der innere Teil einer Filtermembran 13 an dem Führungsdraht 14 befestigt. Die Filtermembran erstreckt sich in der dargestellten Ausführungsform unter einem Winkel von weniger als 90° vom Führungsdraht 14 in radialer Richtung. Der äußerer Bereich der Filtermembran 13 hingegen ist annähernd parallel zur Achse des Führungsdrahtes 14 ausgerichtet. Im äußeren Bereich ist weiterhin eine Schirmstruktur 12 vorgesehen. In der dargestellten Ausführungsform ist die Schirmstruktur 12 in Zick-zack entlang dem äußeren Umfang der Filtermembran 13 und dazu parallel im äußeren Bereich der Filtermembran 13 vorgesehen. Die in Figur 2 dargestellte Position zeigt den Schirm 11 in seiner aufgespannten Form.

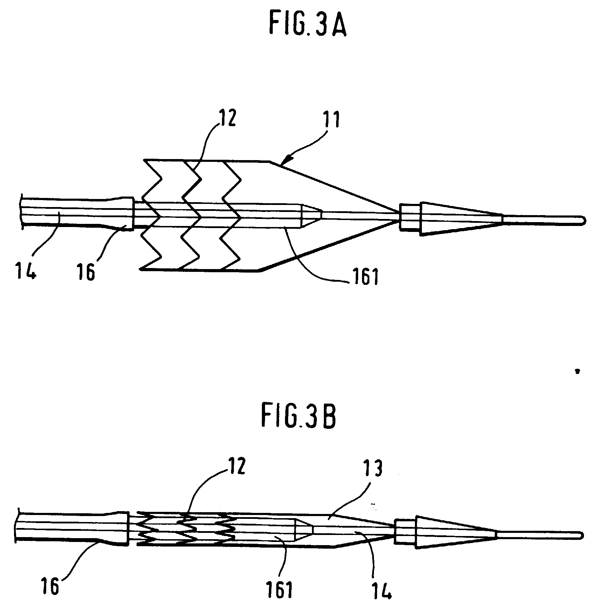

In Figur 3a ist einer Ausführungsform der Erfindung mit Magnetkatheter 16 mit aufgespanntem Schirm 11 schematisch dargestellt. Auf den Führungsdraht 14 ist ein Magnetkatheter 16 aufgefädelt. Dieser weist an seinem dem Schirm 11 zugewandten Ende einen Magneten 161 auf. Bei dieser Ausführungsform besteht die Schirmstruktur 12 aus einem magnetisierbaren Material. Wird der Magnetkatheter 16 soweit in den Schirm 11 geschoben, daß das Material der Schirmstruktur 12 mit dem Magneten 161 in Wechselwirkung treten kann so wird die Schirmstruktur 12 magnetisiert und der Schirm 11 durch die magnetische Wirkung geschlossen. Hierbei bilden also sowohl der auf dem Führungsdraht 14 geführte Magnetkatheter 16 als auch das Material der Schirmstruktur 12 das Mittel zum Schließen des Schirmes 11. Die geschlossene Position des Schirmes 11 ist in Figur 3b dargestellt.

In dieser Position kann der Schirm 11 zusammen mit dem Magneten 161 in eine Führungshülse (nicht dargestellt) zurückgezogen werden. Führungshülse und Schirm können so sicher aus dem Blutgefäß entfernt werden.

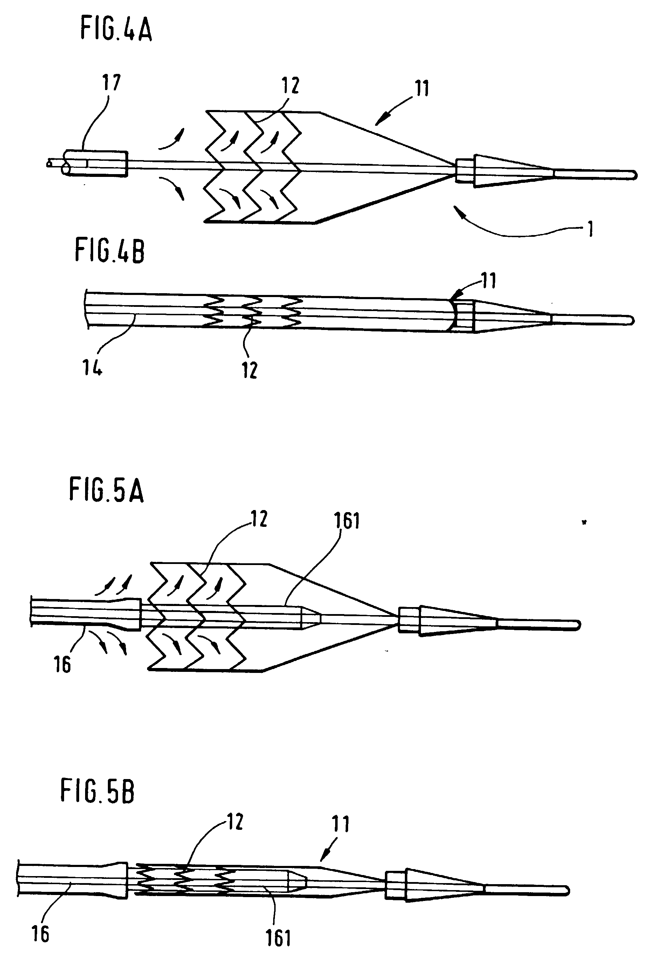

In Figur 4a ist eine weitere Ausführungsform des erfindungsgemäßen Gefäßfiltersystems 1 dargestellt. Bei dieser. Ausführungsform wird die Schirmstruktur 12 durch temperaturempfindliches Material gebildet. Diese Material zieht sich bei Temperaturerniedrigung zusammen. Wird nun wie in Figur 4a durch die Pfeile angedeutet über einen Katheter 17 ein kaltes Medium, z.B. Eiswasser in den Bereich der Schirmstruktur 12 gebracht, so zieht sich diese zusammen und bewirkt das Schließen des Schirmes 11. Die Zufuhr das kalten Mediums wird solange beibehalten, bis der Schirm 11 die in Figur 4b gezeigte Position eingenommen hat. In dieser Position kann der Schirm 11 in eine Führungshülse (nicht dargestellt) gezogen und mit dieser aus dem Blutgefäß entfernt werden.

In Figur 5a ist eine Ausführungsform gezeigt, bei der sowohl ein Magnetkatheter 16 als auch die Zufuhr über einen hier nicht gezeigten weiteren Katheter 17 erfolgen kann. Bei dieser Ausführungsform wird die Schirmstruktur 12 durch Material gebildet, das zum einen temperaturempfindlich und zum anderen magnetisierbar ist. Es ist aber auch möglich, die Schirmstruktur 12 aus zwei Materialien, die entweder miteinander verarbeitet sind oder in separaten Fäden oder Federn vorliegen, herzustellen, wobei jedes der Materialien eine der genannten Eigenschaften aufweist. Bei dieser Ausführungsform wird die Wirkung sowohl des Mittels, das durch das temperaturempfindliche Material dargestellt ist als auch, die Wirkung des Zusammenwirkens von Magnet 161 und zumindest einem Teil der Schirmstruktur 12 genutzt. In Figur 5b ist diese Ausführungsform in dem Zustand des geschlossenen Schirmes 11 dargestellt.

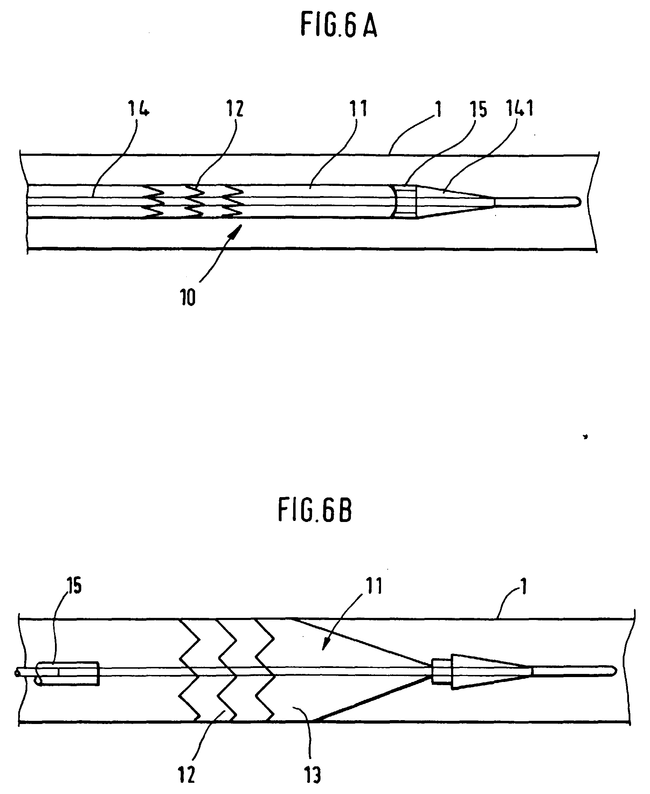

In Figur 6a ist der Zustand gezeigt, in dem ein Gefäßfiltersystem 10 ohne Magnetkatheter in das Blutgefäß 1 eingeführt und auch wieder entfernt wird.

Die Führungshülse 15 umschließt den Schirm 11 und erstreckt sich bis zu dem Aufsatz 141 des Führungsdrahtes 14. Wird die Führungshülse 15 nun bis über den Schirm 11 zurück gezogen, so kann sich dieser Öffnen. Abhängig von dem Material der Schirmstruktur 12 öffnet sich der Schirm 11 aufgrund des Formgedächnisses des Materials, oder aufgrund von Federkraft. Auch eine Kombination dieser beiden Ursachen ist denkbar. Nach dem Öffnen des Schirmes 11 ist der in Figur 6b gezeigte Zustand gegeben. Die Führungshülse 15 befindet sich ― von der Spitze des Führungsdrahtes 14 aus betrachtet - hinter dem Schirm 11. Dieser liegt an der Wand des Blutgefäßes 1 an und verhindert so das Passieren von Thromben neben dem Filter.

Nach Abschluß der Behandlung kann durch die Führungshülse 15 Medium in den Bereich des Schirmes gebracht werden, oder das Material der Schirmstruktur 12 anderweitig, z.B. durch Strahlungen aktiviert werden. Hierdurch wird das Zusammenziehen des Schirmes 11 bewirkt. Bei der in den Figuren gezeigten Ausführungsform des Filters wird sich der äußere Bereich der Filtermembran 13 zusammenziehen. Hierdurch wird ein Herauspressen von im Schirm 11 gefangenen Thromben vermieden.

Der Schirm 11 kann außer der Schirmstruktur 12 im äußeren Bereich auch noch eine weitere Schirmstruktur 12 aufweisen, die sich in Form von Streben vom Führungsdraht 14 zu dem äußeren Bereich der Filtermembran 13 erstreckt. Durch diese Streben wird gewährleistet, daß der Schirm 11 eine gewisse Stabilität aufweist und nach dem Zusammenziehen nahe an dem Führungsdraht 14 oder gegebenenfalls dem Magnetkatheter 16 anliegt und so ohne Hindernisse in die Führungshülse 17 gezogen werden kann.

Nach dem Zurückziehen weist das Gefäßfiltersystem 10 wieder die in Figur 6a dargestellte Form auf und kann leicht aus dem Blutgefäß 1 entfernt werden.

Im Sinne dieser Erfindung wird als Filtermembran das Filtermaterial bezeichnet. Mögliche Filtermembranen sind Netzte oder Geflechte, sowie andere poröse Materialien, die eine ausreichende Flexibilität aufweisen um das Öffnen und Schließen des Schirmes ohne Beschädigung des Materials zu erlauben. Das Material kann textiler Natur sein. Es liegt auch im Sinne der Erfindung die Filtermembran aus einem magnetisierbaren Material zu fertigen. Hierbei wird der Vorgang des Schließens des Schirmes noch unterstützt. Beispielsweise können Metallnetze oder -geflechte eingesetzt werden.

Die Form des Schirmes erstreckt sich im aufgespannten Zustand vorzugsweise im wesentlichen in der Richtung parallel zur Achse des Führungsdrahtes, d.h. in Längsrichtung. Es können insbesondere strumpfförmige Schirme verwendet werden, die im inneren Bereich der Filtermembran Partikel aufnehmen können. Hierdurch wird die Gefahr des Herauspressens von Partikeln beim Schließen verringert.

Für die Schirmstruktur können je nach Ausführungsform magnetisierbare oder temperaturempfindliche Materialien verwendet werden. Insbesondere werden selbstexpandierende Federn aus diesem Material bevorzugt. Es können insbesondere Materialien mit Formgedächnis eingesetzt werden.

Die Form der Schirmstruktur kann unterschiedlich gestaltet sein. Es können Drähte, vorzugsweise mit Federwirkung, aber auch Bänder des Materials eingesetzt werden. Neben der in den Figuren vorgesehenen Form kann die Schirmstruktur auch durch Stege, die sich über die Fläche der Filtermembran vom Führungsdraht zum äußeren Umfang erstrecken dargestellt werden. Auch spiralförmige Anordnungen der Schirmstruktur können im Rahmen der Erfindung verwirklicht werden. Wesentlich für die Erfindung ist, daß die Schirmstruktur zumindest im äußeren Bereich der Filtermembran vorliegt. Durch das Vorsehen der Schirmstruktur in diesem Bereich kann das zuverlässige Schließen des Schirmes bewirkt und ein Entweichen von Partikeln aus der Filtermembran vermieden werden.

Als Führungsdraht können sämtliche im Bereich der Medizin für diese Zwecke bekannten Drähte eingesetzt werden.

Die Abmessungen des Gefäßfiltersystem bestimmen sich nach dem beabsichtigten Verwendungszweck, z.B. dem Durchmesser des zu behandelnden Blutgefäßes. Der Führungsdraht kann beispielsweise einen Durchmesser von 0,014 bis 0,035 inch aufweisen. Die Führungshülse kann einen Durchmesser von beispielsweise 1,67 bis 3,33 mm (5 bis 10 Fr) aufweisen. Der Durchmesser des aufgespannten Schirmes kann beispielsweise 3 bis 10 mm betragen und der Schirm kann in geschlossenem Zustand eine Länge von beispielsweise 10-50 mm haben. Der gegebenenfalls vorgesehene Bereich des Schirmes mit dem dieser an der Wand des Blutgefäßes anliegt kann z.B. 5-20 mm lang sein.

## Patentansprüche

1. Ein Gefäßfiltersystem für die nicht-permanente Anwendung, das umfaßt:
einen Führungsdraht,
eine Filtermembran, die einen im Bereich des proximalen Endes des Führungsdrahtes angebrachten inneren Bereich und einen freien äußeren Bereich aufweist, und
eine Schirmstruktur, die zumindest am äußeren Bereich der Filtermembran vorgesehen ist, aus einem flexiblen Material besteht und das Öffnen und Schließen des durch die Schirmstruktur und die Filtermembran gebildeten Schirmes zuläßt
dadurch gekennzeichnet, daß
das System berührungslos wirkende Mittel zum Schließen des Schirmes aufweist.

2. Gefäßfiltersystem nach Anspruch 1, dadurch gekennzeichnet, daß das Mittel zum Schließen des Schirmes durch das Material der Schirmstruktur gegeben sind.

3. Gefäßfiltersystem nach einem der Ansprüche 1 und 2, dadurch gekennzeichnet, daß zusätzlich ein Mittel zum Schließen des Schirmes auf dem Führungsdraht vorgesehen sind.

4. Gefäßfiltersystem nach einem der vorstehenden Ansprüche, dadurch gekennzeichnet, daß das auf dem Führungsdraht vorgesehene Mittel einen Magnetkatheter darstellt.

5. Gefäßfiltersystem nach einem der vorstehenden Ansprüche, dadurch gekennzeichnet, daß das Material der Schirmstruktur durch Temperaturänderung aktivierbar ist.

6. Gefäßfiltersystem nach einem der vorstehenden Ansprüche, dadurch gekennzeichnet, daß die Schirmstruktur aus Nitinol-Feder gebildet ist.

7. Gefäßfiltersystem nach einem der vorstehenden Ansprüche, dadurch gekennzeichnet, daß das Material der Schirmstruktur magnetisierbar ist.

8. Gefäßfiltersystem nach einem der vorstehenden Ansprüche, dadurch gekennzeichnet, daß das Filtersystem eine Führungshülse zur Aufnahme des Führungsdrahtes und des Schirmes aufweist.
